Europäisches Patentamt

European Patent Office

Office européen des brevets

(11) Numéro de publication: **0 096 134**
**B1**

(12) **FASCICULE DE BREVET EUROPEEN**

(45) Date de publication du fascicule du brevet: **06.11.85**

(21) Numéro de dépôt: **82401055.7**

(22) Date de dépôt: **10.06.82**

(51) Int. Cl.⁴: **A 61 L 2/20**

(54) **Procédé de stérilisation à froid par le formaldéhyde.**

(43) Date de publication de la demande:
**21.12.83 Bulletin 83/51**

(45) Mention de la délivrance du brevet:
**06.11.85 Bulletin 85/45**

(84) Etats contractants désignés:
**BE CH DE GB IT LI NL**

(56) Documents cités:
**DE-A-1 642 095**
**DE-A-2 921 915**
**FR-A-2 079 415**
**FR-A-2 352 551**
**FR-A-2 405 715**
**FR-A-2 502 498**

(73) Titulaire: **DETEC S.A.**
**Rue de Candolle 14**
**CH-1205 Geneve (CH)**

(72) Inventeur: **Brandt, Maurice**
**Rue de Candolle 14**
**CH-1205 Geneve (CH)**

(74) Mandataire: **Flechner, Willy et al**
**CABINET FLECHNER 22, Avenue de Friedland**
**F-75008 Paris (FR)**

Courier Press, Leamington Spa, England.

## Description

La présente invention concerne les procédés de stérilisation par les vapeurs de formaldéhyde, d'objets et d'appareils divers et, notamment, d'objets utilisés en milieu médical ou paramédical, sur lesquels peuvent se fixer des germes pathogènes et qui nécessitent donc des stérilisations régulières. Ces objets sont notamment des instruments chirugicaux, des bassins de lit, mais aussi d'autres objets de la vie courante utilisés dans les höpitaux.

On connaît déjà des procédés d'élimination des germes retenus sur des objets par action d'un mélange de formaldéhyde et de vapeurs d'eau, ou par action du formaldéhyde seul. De tels procédés sont par exemple décrits dans FR—A—2405715 et DE—A—2921915. Dans les deux cas, il faut procéder à des températures de 70°C environ. Certains objets, notamment en matière plastique, ne doivent pas être portés à une température aussi élevée sous peine de se déformer ou d'être détériorés et d'être inaptes ensuite à l'usage auquel ils sont destinés. Un procédé de stérilisation à froid est connu par DE—A—1642095.

L'invention remédie à cet inconvénient par un procédé de stérilisation d'un objet dans une enceinte qui s'effectue sans envoi de vapeur d'eau en grande quantité et à une température relativement basse, comprise notamment entre 30 et 32°C. Malgré cette température relativement basse, le procédé est très efficace et aboutit à une véritable stérilisation de l'objet se trouvant dans l'enceinte.

Le procédé de sterilisation à froid à l'aide de formaldéhyde suivant l'invention consiste à effectuer les stades successifs suivants, de préférence sans attendre entre eux.

a) mettre l'enceinte sous dépression, à température ambiante;

b) y porter le degré hygrométrique à une valeur supérieure à 85%;

c) y abaisser le degré hygrométrique à une valeur inférieure à 75% environ en en portant la température entre 30 et 32°C environ; et

d) y envoyer des vapeurs de formaldéhyde, tout en y maintenant la température entre 30 et 32°C environ.

La succession de ces stades permet d'obtenir la stérilisation de l'objet.

De préférence, on porte le degré hygrométrique au stade b) à une valeur de 90% environ pour abaisser ensuite le degré hygrométrique au stade c) à une valeur de 70% environ.

Suivant une variante particulièrement recommandée, on entrecoupe l'envoi de formaldéhyde de stades brefs de mise sous dépression de l'enceinte. Ces stades brefs de mise sous dépression, tout comme le stade initial de mise sous dépression, ont pour résultat d'obtenir ensuite, lorsque les vapeurs de formaldéhyde sont envoyées dans l'enceinte, qu'elles arrivent même dans les recoins les plus reculés de l'objet.

En portant le degré hygrométrique à une valeur de 90% environ, avant d'envoyer le formaldéhyde dans l'enceinte, on sensibilise les micro-organismes à l'action ultérieure du formaldéhyde.

Chaque mise en dépression dure, en général, de 3 à 10 secondes environ, par exemple 5 secondes, tandis que l'on maintient en général l'hygrométrie de l'enceinte à 90%, c'est-à-dire au-delà de 85% environ pendant 2 à 20 secondes environ, la durée pour parvenir à cette hygrométrie à partir de l'hygrométrie normale initiale de l'enceinte pouvant être d'un quart d'heure environ.

La pression qui règne dans l'enceinte lors d'une dépression est en général inférieure à 100 mbars et peut être égale par exemple à 15 mbars environ.

De préférence on réalise l'abaissement du degré hygrométrique de 90% à 70% par une mise sous dépression de l'enceinte.

On interrompt l'émission des vapeurs de formaldéhyde pendant l'établissement des dépressions mais ensuite, alors que la concentration de formaldéhyde dans l'enceinte correspond à celle qui est nécessaire à la stérilisation, on laisse le formaldéhyde en contact prolongé avec les objets dans l'enceinte, par exemple pendant 1 heure, puis les vapeurs de formaldéhyde contenues dans l'enceinte sont évacuées à l'extérieur par une circulation d'air chauffé à 40°C environ. Les résidus sont ensuite neutralisés par l'ammoniac. Une seconde évacuation créée par une circulation d'air chaud élimine toute trace de vapeur neutralisée formée par le mélange du formaldéhyde et de l'ammoniac.

Aux dessins annexés, donnés uniquement à titre d'exemple:

la figure 1 est un schéma illustrant les diverses fonctions réalisées par l'appareil;

la figure 2 est une vue en perspective avec arrachement partiel d'une enceinte permettant la mise en oeuvre du procédé suivant l'invention; et

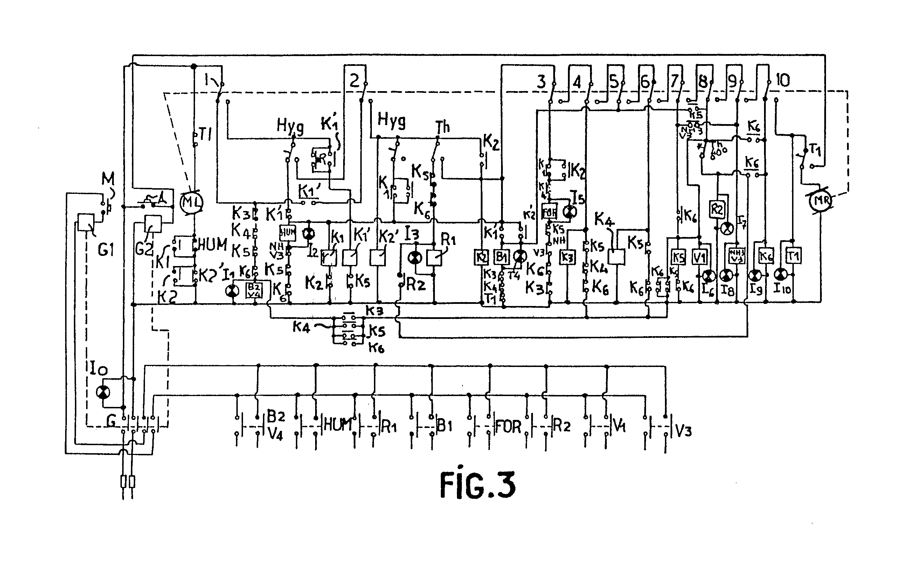

la figure 3 est un schéma électrique de commande et de signalisation des divers organes composant l'appareil.

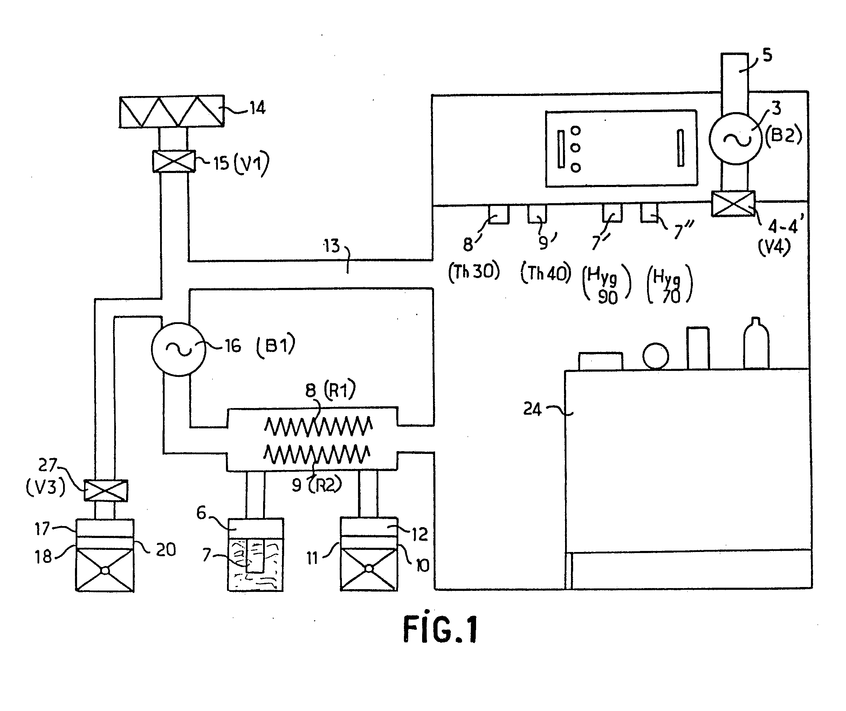

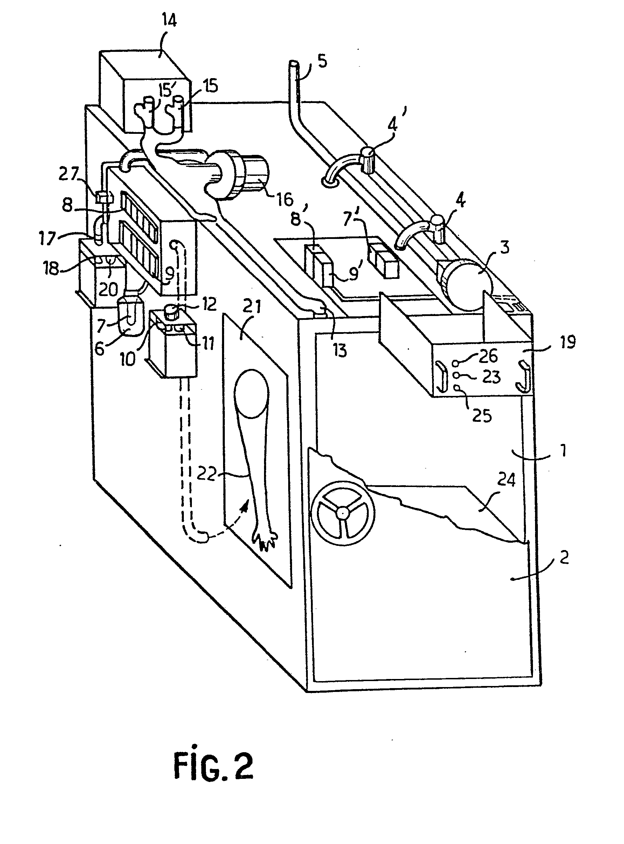

Selon les figures 1 et 2, on voit que l'installation destinée à la stérilisation à froid d'objets divers, et notamment d'appareillages médicaux, se compose d'une enceinte 1, de forme générale parallélépipédique et délimitée par trois parois latérales, un fond ou plancher, un plafond, et une porte 2 d'accès.

L'appareil, ou les objets, soumis à la stérilisation à froid, sera donc conservé pendant le temps nécessaire à l'action de l'agent de stérilisation au sein de l'enceinte constituée par le caisson 1.

De façon à faciliter la pénétration profonde des vapeurs de formaldéhyde dans l'enceinte de stérilisation, la pompe de type pompe à vide 3, est mise en fonction. L'air est aspiré dans l'enceinte 1 à travers les électrovannes 4 et 4', et dégagé vers l'extérieur par la gaine 5.

Dans un second temps, pour obtenir le degré hygrométrique voulu, au sein de l'enceinte 1, un organe d'ajustement du taux d'humidité, par exemple, sous forme d'un conteneur 6 soumis à une résistance chauffante 7, permet de maintenir le degré hygrométrique voulu au sein de l'enceinte; les résistances 8 et 9 sont soumises à une régulation thermostatique 8' et 9', ainsi qu'on l'exposera ci-après, dans la description de la partie électrique. L'humidité relative est également régulée par 7', au cours de cette phase à 90%. Afin d'amener rapidement le degré d'humidité relative à 70%, et la température à 30°C, l'on procède à nouveau à une aspiration de l'air de l'enceinte au moyen de la pompe à vide 3; l'air est rejeté vers l'extérieur, par la gaine 5. Ces conditions permettent d'éviter la repolymérisation du paraformaldéhyde, et rendent optimale l'efficacité des vapeurs de formaldéhyde.

Au sein de l'enceinte 1, le dégagement des vapeurs d'aldéhyde est obtenu par l'action de la résistance 10, formant une plaque chauffante et permettant l'élévation thermique à la température régulée par le doseur d'énergie 11, du bêcher 12, contenant du paraformaldéhyde, qui se sublime sous l'action de l'élévation thermique de la température.

Une gaine d'insufflation d'air 13 pénètre dans l'enceinte 1, et elle permet l'adjonction, au sein de cette enceinte, d'un air destiné à assurer une surpressurisation de l'enceinte à la fin du cycle de stérilisation.

A cet effet, lorsque l'action des vapeurs de formaldéhyde s'est prolongée pendant le temps qui a été programmé et qui correspond aux conditions optimales de stérilisation de l'appareil ou des objets mis en place dans l'enceinte, l'air sous pression est injecté à travers le filtre 14, et les électrovannes 15 et 15'; afin d'éviter une baisse de température au sein de l'enceinte qui risquerait d'entraîner la repolymérisation du paraformaldéhyde, et afin de faciliter l'évacuation de celui-ci, la gaine 13 est associée à une résistance 9, qui assure l'élévation de la température de l'air de rinçage injecté au sein de l'enceinte, en l'amenant à une température voisine de 40°C; cet air provient d'une turbine 16, et il est purifié par passage sur un filtre 14, puis sur deux électrovannes 15 et 15'.

En fin du cycle de stérilisation, dans le cas où les objets à traiter absorbent particulièrement les vapeurs de formaldéhyde, celles-ci sont alors soumises à une opération de neutralisation.

La surpression provoquée au sein de l'enceinte 1 permettra de refouler vers l'extérieur de l'installation, les vapeurs contenues dans l'enceinte 1; la pompe à vide 3, montée sur la canalisation 5, permet, par conséquent, d'aspirer les vapeurs depuis l'enceinte 1, pour les envoyer vers l'extérieur.

A proximité des résistances 8 et 9, se trouve positionnée la source des vapeurs d'ammoniac.

Il s'agira de préférence d'un conteneur, par exemple du type bêcher 17, contenant une solution aqueuse d'ammoniaque et associée à une résistance chauffante 18, dont l'élévation de température est régulée par un doseur d'énergie 20. Les vapeurs d'ammoniac sont transmises à l'enceinte de stérilisation à travers l'électrovanne 27.

Les vapeurs d'ammoniac ainsi dégagées se mélangeront aux vapeurs de formaldéhyde dans l'enceinte 1, puis les vapeurs neutres seront évacuées vers l'extérieur par l'intermédiaire de la gaine 5, et des électrovannes 4 et 4', au cours d'un nouveau cycle de rinçage effectué dans les mêmes conditions que le premier.

La porte 2 est pourvue de joints d'étanchéité, afin d'éviter toute perte ou fuite de formaldéhyde, à l'intérieur de l'enceinte, vers l'extérieur.

Un hublot 21 disposé sur un des côtés de l'enceinte, ou sur la porte avant permet de surveiller l'espace intérieur de l'enceinte depuis l'extérieur tandis qu'un dispositif du type "gant retourné" 22, permet également une intervention de manipulation à l'intérieur du caisson depuis l'extérieur.

Un tableau de commande 19 permet d'afficher et de commander les différentes phases successives du fonctionnement de l'appareil en appuyant sur la touche de commande 23. La touche 25 permet d'arrêter l'appareil en cas d'urgence, la touche 26 accélère le cycle dans les conditions décrites, suivant le schéma électrique. Les objets destinés à la stérilisation, par exemple des instruments chirurgicaux, sont introduits par la porte 2, et déposés sur un support 24, à l'intérieur de l'enceinte; la porte 2 est refermée et le cycle de l'établissement du vide s'effectue pendant une brève période préréglée suivie de la phase de la régulation hygrométrique, qui amène l'atmosphère de l'enceinte 1 au degré hygrométrique voulu; par exemple, à un degré hygrométrique de l'ordre de 90%; ces conditions visant à rendre les germes plus vulnérables à l'action des vapeurs de formaldéhyde.

On procède ensuite à nouveau à l'établissement d'un léger vide de l'atmosphère de l'enceinte de façon à l'amener rapidement à un degrée hygrométrique régulé de l'ordre de 70%, simultanément la température à l'intérieur de l'enceinte est amenée à environ 30°C, également régulée. Ces conditions, évitant la polymérisation du formol, seront conservées pendant la sublimation du paraformaldéhyde et pendant la période de contact.

La phase suivante correspond au dégagement des vapeurs de formaldéhyde au sein de l'enceinte, interrompu par de brèves aspirations de l'atmosphère de celle-ci effectuées par la pompe à vide 3, qui refoule les vapeurs vers l'extérieur de l'installation par l'intermédiaire de la gaine 5.

Cette suite de brèves vidanges de l'air sous pression s'effectue uniquement pendant la phase de sublimation du paraformaldéhyde (alors que la sublimation est interrompue momentanément), et est destinée à faciliter la pénétration des vapeurs d'aldéhyde dans les cavités et pour des objets à stériliser.

Pendant la période de sublimation du paraformaldéhyde et du contact des vapeurs de formaldéhyde avec les objets à stériliser, la turbine 16 est en fonctionnement, ce qui crée un courant intérieur, provoquant le passage régulier des vapeurs de formol recyclées au sein des canalisations internes et la balayage des surfaces des appareils ou des objets à stériliser.

En fin de cycle de stérilisation, les vapeurs de formaldéhyde sont refoulées vers l'extérieur de l'installation par l'intermédiaire de la gaine 5, soit par surpression provoquée par l'injection d'air chaud depuis la gaine 13, soit par action de la pompe 3, aspirant les vapeurs de formaldéhyde depuis l'enceinte 1, pour les rejeter vers l'extérieur.

Dans le cas où il s'agit de stériliser des matériaux absorbant particulièrement les vapeurs de formaldéhyde, on procède ensuite à la neutralisation des vapeurs de formol résiduelles, par l'émission dans l'enceinte des vapeurs d'ammoniac, à faible dose. Pendant cette période, la turbine 16 recycle l'air de l'enceinte, en circuit fermé, ce qui améliore le mélange des vapeurs.

Dans l'ultime phase, on procède à un second rinçage de l'atmosphère de l'enceinte dans les mêmes conditions que le premier, ainsi toute trace de vapeurs de formaldéhyde ou d'ammoniac est définitivement éliminée.

Par action sur le bouton de marche M, le télérupteur G 1 permet le fonctionnement du contacteur général G, ce dernier permet l'enclenchement du contacteur B2 V4, et par conséquent la mise sous tension de la pompe à vide B2 qui aspire l'air à l'intérieur de l'enceinte 1, pour le refouler à l'extérieur de l'installation par l'intermédiaire des électrovannes V4, qui s'ouvrent.

Dès la mise en marche, le moteur ML du programmateur qui va actionner successivement ses 10 cames, est mis en route. La rotation des cames est temporisée en fonction des phases.

Au bout de quelques secondes, la came 1 bascule son contact électrique, ce qui provoque:

— le déclenchement du contacteur B2 V4, donc l'arrêt de la pompe B2, et la fermeture de l'électrovanne V4;
— la mise en circuit de l'humidificateur HUM, par l'enclenchement du contacteur correspondant. La vapeur d'eau est brumisée dans l'enceinte jusqu'à atteindre les 90% d'humidité relative programmés sur l'hygrostat HYG/90;
— simultanément, le relais K1 enclenche, et isole l'hygrostat HYG/70, et le contacteur FOR;
— un contact de HUM arrête le moteur ML du programmateur, car la durée de la montée en hygrométrie dans l'enceinte dépend de l'humidité relative initiale et ne peut être temporisée;
— la turbine de recyclage B1 est mise en service et accélère la montée en hygrométrie dans l'enceinte.

Lorsque le degré hygrométrique 90 est atteint, le contact électrique de l'hygrostat HYG/90 s'inverse, ainsi:

— le relais K1 enclenche et est auto-alimenté par un des ses contacts, le relais K1 déclenche, ce qui provoque:
  . l'enclenchement du contacteur B2 V4, qui met à nouveau en service la pompe à vide B2, qui aspire l'air à l'intérieur de l'enceinte pour le refouler à l'extérieur par l'intermédiaire des électrovannes V4 qui s'ouvrent;
  . la turbine B1 s'arrête;
  . le moteur ML du programmateur reprend son cycle.

Après quelques secondes, la came 2 du moteur ML bascule son contact; le relais K2' enclenche, et arrête à nouveau le moteur ML. Le relais K2' remet en fonctionnement la turbine B1 qui brasse dans l'enceinte en circuit fermé, la pompe à vide B2 étant arrêtée, et les électrovannes V4 fermées, un air chauffé et humidifié destiné à mettre en condition l'atmosphère de l'enceinte avant la sublimation du formaldéhyde.

En effet, l'humidificateur HUM est remis en circuit et commandé par l'hygrostat HYG/70, jusqu'à obtenir 70% d'humidité relative. Simultanément, le contacteur R1 enclenche et met sous tension les résistances chauffantes R1, commandées par le thermostat TH 30, qui amènent rapidement la température ambiante de l'atmosphère de l'enceinte à 30°C.

Lorsque cette température est atteinte, le contact électrique du thermostat TH 30 bascule et provoque l'enclenchement du relais K2, qui est autoalimenté, et remet en fonctionnement le moteur ML du programmateur. Le contacteur FOR s'enclenche, la sublimation du formaldéhyde commence. L'enceinte 1 est balayée par un air chauffé et humidifié et par les vapeurs de formeol, la phase de stérilisation commence.

Au bout de 5 minutes, la came 3 du programmateur bascule, ce qui entraîne:

— l'enclenchement de K3;
— l'arrêt momentané de la sublimation du formol par le déclenchement du contacteur FOR;
— l'arrêt de la turbine de recyclage B1;
— à nouveau, la mise sous tension de la pompe à vide B2 qui aspire l'air à l'intérieur de l'enceinte et le rejette à l'extérieur par l'intermédiaire des électrovannes V4 ouvertes, de façon à faciliter la pénétration ultérieure des vapeurs de formaldéhyde par absorption dans les cavités et pour des objets à stériliser.

Pendant la phase de sublimation du formaldéhyde, on procède à deux phases de vide. Après un temps très bref, la came 4 bascule, ce qui provoque:

— le déclenchement du relais K3, l'arrêt de la pompe B2, la fermeture des électrovannes V4, la pompe de recyclage B1 est remise sous tension;
— le contacteur FOR se réenclenche, la sublimation du formaldéhyde se poursuit.

Après 5 minutes, la came 5 bascule à son tour, et le relais K4 enclenche et entraîne:
. l'arrêt de la turbine B1;
. à nouveau la mise sous tension de la pompe à vide B2, qui fonctionne avec les électrovannes V4, dans les mêmes conditions que précédemment. Au bout de quelques secondes, la came 6 bascule, le relais K4 se déclenche, ce qui provoque l'arrêt de la pompe B2, et la fermeture des électrovannes V4, la pompe de recyclage B1 est remise en service; la sublimation du formaldéhyde se poursuit encore quelques minutes.

Puis le temps de contact des vapeurs de formaldéhyde avec les objets à stériliser débute, il se réduira à un temps très court de quelques minutes. La durée de la sublimation du formaldéhyde et du temps de contact est d'environ 1 heure, ainsi la durée totale du cycle n'excède pas 4 heures.

A la fin du temps de contact, la came 7 du programmateur s'inverse. Le relais K5 enclenche, la turbine de recyclage B1 aspire l'air extérieur, à travers le filtre 14 et les électrovannes V1 qui s'ouvrent sous l'action de l'enclenchement du contacteur V1. Simultanément, la batterie chauffante R2 est mise sous tension, avec la R1, par l'enclenchement du contacteur R2 et par conséquent de R1. La température de l'air qui traverse l'enceinte, aspiré par la pompe B2 et rejeté à l'extérieur de l'installation par l'intermédiaire des électrovannes V4, et de la gaine 5, est régulée à 40°C, par le thermostat TH 40.

Ainsi, pendant une heure, les vapeurs de formaldéhyde sont refoulées à l'extérieur de l'enceinte 1.

Au bout de ce temps, la totalité des vapeurs de formaldéhyde est extraite mais dans certains cas où les objets à stériliser sont constitués de matériaux absorbant particulièrement le formaldéhyde, il est nécessaire de neutraliser par une injection dans l'enceinte, d'une très faible dose d'ammoniac, les infimes résidus du formol.

A cet effet, l'appareil est équipé d'une plaque chauffante AMMONIAC, commandée par un contacteur NH3/V3, et régulée en température par un thermostat ou doseur d'énergie. L'évaporation de l'ammoniac étant assez rapide, dans le cas où l'apport de la neutralisation n'est pas nécessaire, le bécher AMMONIAC n'est pas rempli.

A la fin du temps de la première évacuation des vapeurs d'aldéhyde, la came 8 du programmateur bascule son contact électrique, et le contacteur NH3/V3 enclenche. L'évaporation de l'ammoniac commence, les vapeurs sont transmises dans l'enceinte par l'électrovanne V3. La pompe à vide B2 est arrêtée, et les électrovannes V4 et V1 sont fermées par le déclenchement du relais K5. La turbine B1 crée une circulation d'air chargé de vapeurs d'ammoniac, en circuit fermé, à travers l'enceinte 1. Ainsi, en quelques minutes, les vapeurs de formaldéhyde résiduelles sont neutralisées par les vapeurs d'ammoniac.

A la fin de la neutralisation, la came 9 du programmateur, toujours entraînée par le moteur ML, inverse son contact électrique, le contacteur NH3/V3 déclenche, et arrête le fonctionnement de la plaque chauffante AMMONIAC.

Il est nécessaire de façon à éliminer les vapeurs neutralisées que représente le mélange des gaz formaldéhyde et ammoniac, de procéder en fin de cycle, à une seconde évacuation par rinçage à l'air chaud, de l'enceinte de stérilisation et de ses canalisations.

Pendant les deux phases d'évacuation, l'humidificateur est mis hors service car le rinçage est plus efficace avec un air sec. Il en est de même, lors de la phase ammoniac, car l'évaporation de ce gaz s'accompagne d'un dégagement de vapeurs d'eau et par conséquent d'une élévation du degré hydrométrique.

Ainsi, le contacteur K6 enclenche et provoque:

— le fonctionnement de la pompe à vide B2;
— l'ouverture des électrovannes V4 et V1;
— le chauffage de l'air de rinçage de l'enceinte par la mise en service des batteries de résistances R1 et R2, régulées à une température de 40°C, par le thermostat TH 40.

L'aspiration de l'air extérieur à travers le filtre par la turbine B1 se produit dans les mêmes conditions que celles de la première évacuation.

Pendant 15 minutes environ, l'air épuré est aspiré par la turbine B1, à travers l'électrovanne V1 et réchauffé au contact des résistances R1 R2. Simultanément, la pompe B2 aspire dans l'enceinte les vapeurs neutralisées mélangées à l'air chaud, et les relette à l'extérieur de l'installation, au travers des électrovannes V4, par l'intermédiaire de la gaine 5.

Après 15 minutes de rinçage, suffisantes pour éliminer toutes traces de vapeurs résiduelles, la came 10 du programmateur inverse son contact électrique, ce qui provoque:

— le déclenchement du relais K6 qui entraîne:
. l'arrêt des pompe et turbine B2 et B1;
. la fermeture des électrovannes V1 et V4;
. la mise hors-circuit des résistances R1, R2.

Le relais de temporisation à retard à l'ouverture T1 enclenche, et met en service le moteur MR, du programmateur qui, en 10 secondes, ramène les 10 cames du programmateur à leur position initiale, du début de cycle. Simultanément, le moteur ML est arrêté par la coupure d'un contact du relais temporisateur T1.

Après 10 secondes, le contact temporisé du relais T1 bascule et enclenche le télérupteur G2, qui ouvre le contacteur général G. La coupure du courant ramène les contacts du relais temporisé T1, à leur position initiale.

Le cycle est terminé, la porte 1 est déverrouillée, les objets sont retirés stérilisés de l'enceinte.

Le schéma électrique est représenté à titre d'exemple et peut être simplifié par l'emploi de composants électriques tels que les microprocesseurs. En cas de nécessité, le cycle est interrompu manuellement, et ramené à sa position de départ, par l'enclenchement de la touche A. Si le cycle est interrompu accidentellement (tel par exemple que l'arrêt consécutif à une coupure de courant), après la phase d'hygrométrie à 90% d'humidité, l'utilisateur a la possibilité, en appuyant sur la touche R qui provoque l'enclenchement du relais K1, de pouvoir redémarrer le cycle à l'endroit où il s'était arrêté mais, après que la période de préchauffage se soit écoulée, de façon à éviter la polymérisation du paraformaldéhyde.

Dans le cas de l'appareil avec composants électroniques, dès la remise sous tension, le cycle reprendra automatiquement à l'endroit où il s'était interrompu, grâce à un circuit à mémoire, mais également, si nécessaire, après que la période de préchauffage se soit écoulée.

## Revendications

1. Procédé de stérilisation à froid à l'aide de formaldéhyde d'un objet se trouvant dans une enceinte, caractérisé en ce qu'il consiste à effectuer les stades successifs suivants:

a) mettre l'enceinte sous dépression, à température ambiante;

b) y porter le degré hygrométrique à une valeur supérieure à 85%;

c) y abaisser le degré hygrométrique à une valeur inférieure à 75% environ en en portant la température entre 30 et 32°C environ; et

d) y envoyer des vapeurs de formaldéhyde, tout en y maintenant la température entre 30 et 32°C environ.

2. Procédé suivant la revendication 1, caractérisé en ce qu'il consiste à effectuer les stades a) à d) sans attente entre eux.

3. Procédé suivant la revendication 1 ou 2, caractérisé en ce qu'il consiste à porter l'hygrométrie de l'enceinte à 90% environ du stade b).

4. Procédé suivant l'une des revendications précédentes, caractérisé en ce qu'il consiste à abaisser le degré hygrométrique de l'enceinte à 70% environ au stade c).

5. Procédé suivant l'une des revendications précédentes, caractérisé en ce qu'il consiste à entrecouper l'envoi de formaldéhyde de stades brefs de mise sous dépression de l'enceinte.

6. Procédé suivant l'une des revendications précédentes, caractérisé en ce qu'il consiste à maintenir l'hygrométrie de l'enceinte au-delà de 85% environ pendant 2 à 20 secondes environ.

7. Procédé suivant l'une des revendications précédentes, caractérisé en ce que la ou les dépressions durent de 3 à 10 secondes environ.

8. Procédé suivant l'une des revendications précédentes, caractérisé en ce que la pression qui règne dans l'enceinte mise sous dépression est inférieure à 100 mbars.

9. Procédé suivant l'une des revendications précédentes, caractérisé en ce qu'il consiste à faire suivre le stade d) d'envoi de vapeurs de formaldéhyde d'un stade de neutralisation de ces vapeurs, à l'aide d'ammoniac envoyé dans l'enceinte.

## Patentansprüche

1. Verfahren zur Kaltsterilisation eines in einem umschlossenen Raum befindlichen Gegenstandes mittels Formaldehyd, dadurch gekennzeichnet, daß nacheinander die folgenden Verfahrensschritte ausgeführt werden:

a) der umschlossene Raum wird bei Umgebungstemperatur einem Unterdruck ausgesetzt;

b) der Feuchtigkeitsgrad wird darin auf einen Wert über 85% gebracht:

c) der Feuchtigkeitsgrad wird darin auf einen Wert unter etwa 75% abgesenkt und die Temperatur zwischen etwa 30 und 32°C gehalten;

d) es wird Formaldehyddampf zugeführt und die Temperatur zwischen etwa 30 und 32°C aufrechterhalten.

2. Verfahren nach Anspruch 1, dadurch gekennzeichnet, daß die Verfahrensschritte a) bis d) unmittelbar aufeinanderfolgend ausgeführt werden.

3. Verfahren nach Anspruch 1 oder 2, dadurch gekennzeichnet, daß beim Verfahrensschritt b) der Feuchtigkeitsgrad des umschlossenen Raumes etwa 90% beträgt.

4. Verfahren nach einem der vorstehenden Ansprüche, dadurch gekennzeichnet, daß beim Verfahrensschritt c) der Feuchtigkeitsgrad des umschlossenen Raumes etwa 70% beträgt.

5. Verfahren nach einem der vorstehenden Ansprüche, dadurch gekennzeichnet, daß die Zufuhr von Formaldehyd durch kurze Verfahrensschritte unterbrochen wird, in denen der umschlossene Raum einem Unterdruck ausgesetzt wird.

6. Verfahren nach einem der vorstehenden Ansprüche, dadurch gekennzeichnet, daß der Feuchtigkeitsgrad des umschlossenen Raumes über etwa 85% auf die Dauer von etwa 2 bis 20 Sekunden gehalten wird.

7. Verfahren nach einem der vorstehenden Ansprüche, dadurch gekennzeichnet, daß der oder die Unterdrücke etwa 3 bis 10 Sekunden andauern.

8. Verfahren nach einem der vorstehenden Ansprüche, dadurch gekennzeichnet, daß der in dem dem Unterdruck ausgesetzten umschlossenen Raum herrschende Druck unter 100 mbar liegt.

9. Verfahren nach einem der vorstehenden Ansprüche, dadurch gekennzeichnet, daß dem die Zufuhr von Formaldehyddampf betreffenden Verfahrensschritt d) ein Verfahrensschritt zur Neutralisation dieses Dampfes durch Einleiten von Ammoniak in den umschlossenen Raum folgt.

## Claims

1. Process for the cold sterilization with the aid of formaldehyde of an object in an enclosure, characterized in that it comprises performing the following successive stages:

a) placing the enclosure under a vacuum at ambient temperature;

b) raising its relative humidity to a value exceeding 85%;

c) lowering its relative humidity to a value below approximately 75% whilst raising its temperature to between approximately 30 and 32%; and

d) feeding in formaldehyde vapours, whilst maintaining the temperature therein at between approximately 30 and 32°C.

2. Process according to claim 1, characterized in that it consists of performing stages a) to d) without any wait between them.

3. Process according to claims 1 or 2, characterized in that it consists of raising the relative humidity of the enclosure to approximately 90% in stage b).

4. Process according to any one of the preceding claims, characterized in that it consists of lowering the relative humidity of the enclosure to approximately 70% in stage c).

5. Process according to any one of the preceding claims, characterized in that it consists of interspersing the supply of formaldehyde with brief stages during which the enclosure is placed under a vacuum.

6. Process according to any one of the preceding claims, characterized in that it comprises maintaining the relative humidity of the enclosure at above approximately 85% for approximately 2 to 20 seconds.

7. Process according to any one of the preceding claims, characterized in that the vacuum period or periods last approximately 3 to 10 seconds.

8. Process according to any one of the preceding claims, characterized in that the pressure prevailing in the enclosure placed under a vacuum is below 100 mbars.

9. Process according to any one of the preceding claims, characterized in that it consists of following stage d) consisting of feeding in the formaldehyde vapours by a stage comprising the neutralization of these vapours with the aid of ammonia fed into the enclosure.

FIG.1

FIG. 2

FIG.3